# EUROPÄISCHE PATENTANMELDUNG

(11) **EP 2 641 972 A1**
(43) Veröffentlichungstag der Anmeldung: **25.09.2013**
(21) Anmeldenummer: 12160810.3
(22) Anmeldetag: 22.03.2012
(51) Int. Cl.: C12P 13/00

(54) **Verfahren zur asymmetrischen Aminierung von Ketonen unter Verwendung von omega-Transamminasen in organischen Lösungsmitteln**

(71) Anmelder: Universität Graz, 8010 Graz (AT)
(72) Erfinder: Mutti, Francesco, 8010 Graz (AT); Kroutil, Ao. Univ. Prof. DI Wolfgang, 8042 Graz (AT)
(74) Vertreter: Ellmeyer, Wolfgang

(57) **Zusammenfassung**

Die Erfindung betrifft ein Verfahren zur Herstellung eines Amins (3) durch Aminierung eines Ketons (1) mittels enzymatischer Katalyse unter Verwendung einer ω-Transaminase (ω-TA) als Katalysator, von Pyridoxal-5'-phosphat (PLP) als Cofaktor und eines Amins (2) als Amino-Donor, wobei die nachstehende Reaktion in einem organischen Lösungsmittel durchgeführt wird: 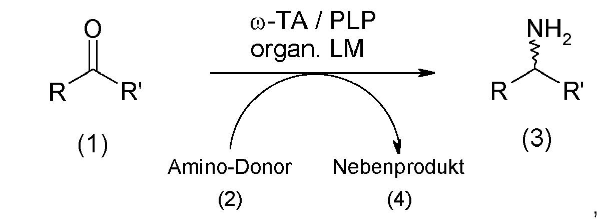 worin R und R' einwertige Kohlenwasserstoffreste sind, mit dem Kennzeichen, dass im organischen Lösungsmittel eine zwischen 0 und der Sättigungskonzentration c_{S} des jeweiligen Lösungsmittels liegende Wasserkonzentration c_{W} eingestellt wird, bevor die Aminierungsreaktion durchgeführt wird, so dass gilt: 0 < _{Cw} < c_{S}.

## Beschreibung

Die Erfindung betrifft ein Verfahren zur asymmetrischen Aminierung von Ketonen in organischen Lösungsmitteln unter Verwendung von ω-Transaminasen.

ω-Transaminasen sind von Pyridoxal-5'-phosphat (PLP) abhängige Enzyme aus den Klassen III und IV der Familie der Aminotransferasen. Bei ihrer enzymatischen Reaktion überträgt PLP als Cofaktor formal Ammoniak und Elektronen von einem Amino-Donor auf ein Keton als Akzeptor. Im Gegensatz zu α-Aminotransferasen können ω-Transaminasen auch Substrate nutzen, die keine Carboxylgruppe in α-Stellung aufweisen, weswegen sie interessante Katalysatoren für die organische Synthese darstellen. Im Vergleich zu üblichen chemischen Methoden ermöglicht die enzymatische Katalyse schonendere Reaktionsbedingungen (z.B. Atmosphärendruck, Raumtemperatur), höhere Enantioselektivität, breite Substratspezifität und den Ausschluss von Verunreinigungen durch Schwermetalle.

Bisher wurden solche Biotransformationen mittels ω-Transaminasen praktisch ausschließlich in wässrigen Medien, d.h. wässrigen Puffern, mitunter in Kombination mit einem Co-Lösungsmittel, durchgeführt, was allerdings zahlreiche Probleme bereitet. So liegt etwa das Gleichgewicht bei der Aminierung von Ketonen unter Verwendung von Alanin als Aminodonor aufseiten des Ketons, wodurch ein großer, z.B. fünffacher, Überschuss an Alanin erforderlich ist. Weiters wäre zwar das achirale und kostengünstige 2-Propylamin ein nahezu idealer Donor, da die enzymatischen Reaktionen jedoch zumeist bei neutralem pH (zwischen pH 6 und 8) durchgeführt werden, wo 2-Propylamin praktisch vollständig protoniert vorliegt, ist erneut ein riesiger Überschuss (zumindest 20fach) des Amins erforderlich. Zudem muss das als Nebenprodukt anfallende Aceton relativ aufwändig aus dem wässrigen System entfernt werden, z.B. durch Spülen des Reaktionsgemischs mit Stickstoffgas oder durch Anlegen von Vakuum.

Folglich wäre es wünschenswert, ein Verfahren zu entwickeln, mit dem diese Probleme gelöst werden können. Im Zuge ihrer Forschungen haben die Erfinder nun herausgefunden, dass die enzymatische Katalyse unter Einsatz von ω-Transaminasen unter bestimmten Umständen in nichtwässrigen Systemen, d.h. in reinen organischen Lösungsmitteln durchgeführt werden kann. Ein derartiges Reaktionssystem wurde bisher nur in einer Publikation beschrieben (siehe Kim et al., Biotechnol. Bioeng. 93(2), 391-5 (2006)). Dabei wurde mittels einer aus einem Zellextrakt gereinigten und lyophilisierten ω-Transaminase in Ethylacetat als Lösungsmittel ein einziges Substrat, nämlich 2-Hydroxy-1-indanon, selektiv zu trans-(1*R*,2*R*)-1-Amino-2-indanol umgesetzt. Allerdings waren die Umsatzzahlen und die Enantiomerenreinheit des Produkts relativ gering, weswegen Aminierungen unter Verwendung von (ω-Transaminasen in organischen Lösungsmitteln später nicht mehr näher untersucht wurden.

Interessant sind jedoch die folgenden drei allgemeinen Beobachtungen der Autoren:
A) Es erfolgt ein Wasseraustausch zwischen dem Lösungsmittel und dem Enzympräparat. Da selbst ein getrocknetes (z.B. lyophilisiertes) Enzympräparat eine gewisse Restmenge an Wasser enthält, stellt sich mit der Zeit ein Gleichgewicht zwischen der festen Enzym- und der flüssigen Lösungsmittelphase ein. Das heißt, dass bei Einsatz eines vollkommen wasserfreien Lösungsmittels Wasser aus dem Enzympräparat in das Lösungsmittel migriert, was dessen Wassergehalt erhöht, während bei Verwendung eines bereits mit Wasser gesättigten Lösungsmittels Wasser an die feste Phase adsorbiert wird, wobei der Wassergehalt des Lösungsmittels abnimmt.
B) Die Reaktivität des Enzyms machte im besten getesteten organischen Lösungsmittel, eben Ethylacetat, nur etwa 30% der Aktivität in einem wässrigen Puffersystem aus.
C) Die Reaktivität des Enzyms war in mehreren getesteten Lösungsmitteln im Vergleich zum jeweiligen mit Wasser gesättigten Lösungsmittel durchwegs niedriger, wenn ein vollkommen wasserfreies Lösungsmittel zum Einsatz kam. Bei Ethylacetat, das aufgrund der höchsten Enzymaktivität für die Synthese eingesetzt wurde, war der Unterschied am stärksten ausgeprägt, wobei die Aktivität des Enzyms mehr als das 100fache jener im wasserfreien Lösungsmittel betrug. Diesen Umstand erklären die Autoren durch die Gegenwart einer größeren Anzahl an Wassermolekülen an der Oberfläche des Enzyms.

Die Lehre des Artikels von Kim et al. lautet demnach, dass enzymatische Aminierungen unter Verwendung von ω-Transaminasen am besten in wässrigen Pufferlösungen durchzuführen sind. Falls stattdessen doch ein organisches Lösungsmittel eingesetzt wird, sollte es mit Wasser gesättigt sein.

Ziel der Erfindung war vor diesem Hintergrund die Entwicklung eines verbesserten Syntheseverfahrens für Amine unter Verwendung von ω-Transaminasen als Katalysatoren in organischen Lösungsmitteln.

### OFFENBARUNG DER ERFINDUNG

Dieses Ziel wird durch Bereitstellung eines Verfahrens zur Herstellung eines Amins (3) durch Aminierung eines Ketons (1) mittels enzymatischer Katalyse unter Verwendung einer ω-Transaminase (ω-TA) als Katalysator, von Pyridoxal-5'-phosphat (PLP) als Cofaktor und eines Amins (2) als Amino-Donor erreicht, wobei die nachstehende Reaktion in einem organischen Lösungsmittel durchgeführt wird: 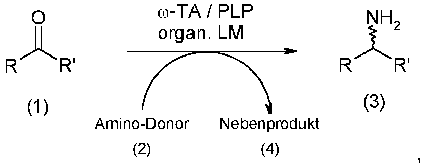 worin R und R' einwertige Kohlenwasserstoffreste sind, das dadurch gekennzeichnet ist, dass im organischen Lösungsmittel eine zwischen 0 und der Sättigungskonzentration cₛ des jeweiligen Lösungsmittels liegende Wasserkonzentration c_{w}eingestellt wird, bevor die Aminierungsreaktion durchgeführt wird, so dass gilt: 0 < c_{w} < cₛ.

Bei näherer Untersuchung der Enzymaktivität von ω-Transaminasen in verschiedensten organischen Lösungsmitteln haben die Erfinder nämlich überraschenderweise festgestellt, dass das Enzym ein vom Wassergehalt des Lösungsmittels abhängiges Aktivitätsmaximum aufweist, das bei einer Konzentration unterhalb des jeweiligen Wassersättigungswerts des Lösungsmittels lag. Diese maximale Aktivität des Enzyms war zudem nicht nur höher als jene bei 0 % Wassergehalt und jene im wassergesättigten Lösungsmittel, sondern mitunter sogar höher als seine Aktivität in einem wässrigen Puffersystem. Folglich können bestimmte organische Lösungsmittel sogar bessere Medien für Transaminierungen als Wasser darstellen.

R und R' sind vorzugsweise aus der aus Alkyl-, Aryl-, Arylalkyl- und Alkylaryl-Resten bestehenden Gruppe ausgewählt, wobei die Reste gegebenenfalls substituiert, insbesondere mit einer oder mehreren Hydroxy-, Oxo-, Carbonyl- und/oder Carboxylgruppen substituiert, sind und/oder gegebenenfalls ein oder mehrere Kohlenstoffatome durch Heteroatome, z. B. O-, N- oder S-, insbesondere O-Atome, ersetzt sind.

In bevorzugten Ausführungsformen weist zumindest einer von R und R' die Struktur -CH₂-O-R" oder -CH₂-(C=O)-R" auf, worin R" ein Wasserstoffatom oder ein einwertiger Kohlenwasserstoffrest wie oben definiert ist, da sich gezeigt hat, dass derart "aktivierte" Ketogruppen besonders gut geeignete Substrate im erfindungsgemäßen Transaminierungsverfahren darstellen.

Für den Fachmann versteht es sich von selbst, dass die optimale Wasserkonzentration für das jeweilige Reaktionsgemisch mittels einfacher Vorversuche mit kleinen Reaktionsansätzen zu bestimmen ist. Der Wassergehalt kann dabei auf beliebige Weise bestimmt werden, z.B. mittels Karl-Fischer-Titration, aber auch durch Messungen physikalischer Größen wie etwa des Brechungsindex des Lösungsmittels.

Die Einstellung der jeweils optimalen Wasserkonzentration im Lösungsmittel kann auf beliebige Weise erfolgen, am einfachsten durch geeignete Wahl der Mengen der Komponenten des Reaktionsgemischs, insbesondere jener des Lösungsmittels und des Enzympräparats. Vorzugsweise wird aber in allen Fällen die ω-Transaminase im Lösungsmittel äquilibrieren gelassen, bis sich eine konstante Wasserkonzentration des Lösungsmittels eingestellt hat. Falls diese noch nicht der optimalen Konzentration für die Aminierungsreaktion entspricht, kann diese durch Zudosieren von Wasser bzw. wasserfreiem Lösungsmittel eingestellt werden. Derartiges Zudosieren kann zu einem Gemisch erfolgen, das nur aus Lösungsmittel und ω-Transaminase besteht, oder zu einem, das auch bereits weitere Reaktionskomponenten, z.B. Cofaktor, Substrat, pH-Regler oder andere, optionale Komponenten, umfasst.

Die jeweils einzusetzende ω-Transaminase ist nicht speziell eingeschränkt. Selbstverständlich sollte in allen Fällen ein Enzym mit hoher Spezifität für das jeweilige Substrat sowie möglichst hohen Wechselzahlen ("turnover numbers") gewählt werden. Dieses kann beispielsweise als Aufschlämmung in einem nichtwässrigen Lösungsmittel, das dasselbe Lösungsmittel wie für die Aminierungsreaktion oder auch ein anderes Lösungsmittel sein. Vorzugsweise wird das Enzym in fester Form, insbesondere in getrockneter, vorzugsweise lyophilisierter, Form eingesetzt. Es kann dabei in Pulverform oder an einen festen Träger immobilisiert vorliegen, beispielsweise an Perlen aus Glas, Kunststoff oder Metall, z.B. an Magnetperlen, gebunden. In den Beispielen als Ausführungsformen der vorliegenden Erfindung hat sich als besonders bevorzugtes Enzym von *Arthrobacter sp.* stammende (R)-selektive ω-Transaminase herausgestellt. Das erfindungsgemäße Transaminierungsverfahren konnte auch mit der ω-TA von *Chromobacterium violaceum,* der ω-TA von *Pseudomonas fluorescens,* der His-markierten ω-TA von *Vibrio fluvialis,* der Strep-markierten ω-TA von *Paracoccus denitrificans,* der ω-TA von *Bacillus megaterium,* der His-markierten, (S)-selektiven ω-TA von *Arthrobacter citreus,* der ω-TA von *Alcaligenes denitrificans* und der (R)-selektiven ω-TA der Mutante 11 von *Arthrobacter sp.* durchgeführt werden, wobei mitunter auch sehr gute Ergebnisse erzielt wurden. Die Erfindung ist aber natürlich nicht auf diese Enzyme beschränkt, da dasselbe Prinzip als für sämtliche ω-Transaminasen anwendbar angesehen wird.

Die Gewinnung des Enzyms, sofern es nicht als Fertigpräparat im Handel erhältlich ist, erfolgt normalerweise durch Kultivierung von Zellen, die in der Lage sind, das Enzym zu produzieren, üblicherweise gefolgt von Zellaufschluss (z.B. durch Beschallung, Behandlung mit Lysozym, French Press usw.), Zentrifugation und Abtrennung des Zellpellets, wobei der Überstand als Zellextrakt zurückbleibt. Dieser kann vor der anschließenden Trocknung weiteren Reinigungsschritten unterzogen werden. In bevorzugten Ausführungsformen wird als ω-Transaminase aber ein lyophilisierter Rohextrakt von das Enzym produzierenden Zellen eingesetzt, so dass aufwändige Isolations- und Reinigungsschritte vor dem Lyophilisieren entfallen können.

Das organische Lösungsmittel ist hierin nicht speziell eingeschränkt, und es kommen jegliche Lösungsmittel in Frage, in denen die Aminierungsreaktion zufrieden stellend durchgeführt werden kann. Dabei kann jeweils ein einzelnes Lösungsmittel oder auch ein Gemisch zweier oder mehrerer Lösungsmittel eingesetzt werden. Sind die Wasserkonzentrationen der im Gemisch enthaltenen Einzellösungsmittel, bei denen das Enzym jeweils sein Aktivitätsmaximum zeigt, bekannt, so kann näherungsweise ein Mittelwert dieser optimalen Wasserkonzentrationen als Ziel-Wasserkonzentration für die Aminierung im Lösungsmittelgemisch herangezogen werden. Es empfiehlt sich jedoch, die optimale Wasserkonzentration für das Gemisch gesondert zu bestimmen, da das Aktivitätsmaximum des Enzyms aufgrund von Wechselwirkungen der Lösungsmittelmoleküle im Gemisch untereinander und mit den übrigen Reaktionskomponenten gegenüber diesem rechnerischen Mittelwert mehr oder weniger stark verschoben sein kann.

Vorzugsweise wird gemäß vorliegender Erfindung ein aus Methyl-tert-butylether, Ethylacetat, Acetonitril, Diisopropylether, Dioxan und Gemischen davon ausgewähltes organisches Lösungsmittel eingesetzt, da diese Lösungsmittel bei den von den Erfindern durchgeführten Aminierungsreaktionen gute Ergebnisse geliefert haben. Unter diesen Lösungsmitteln sind wiederum Methyl-tert-butylether und Ethylacetat zu bevorzugen, wovon Methyl-tert-butylether besonders bevorzugt wird.

### KURZBESCHREIBUNG DER ZEICHNUNG

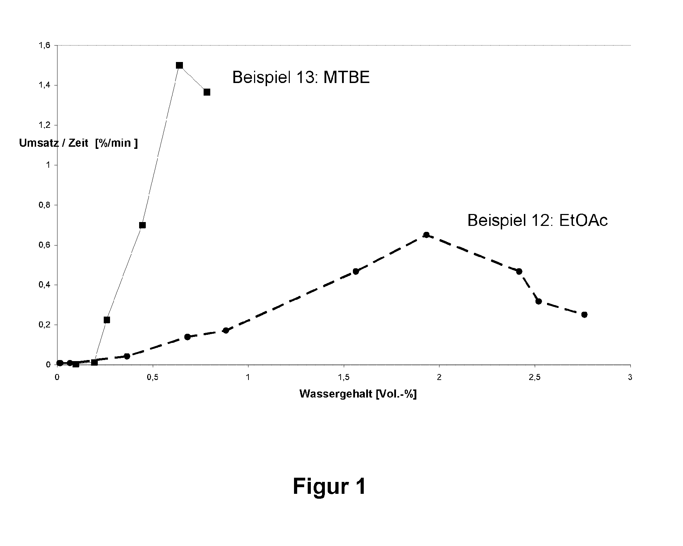

Fig. 1 zeigt die in den Beispielen 12 und 13 bei unterschiedlichen Wassergehalten zweier organischer Lösungsmittel erzielten Umsätze der Transaminierung.

Nachstehend wird die vorliegende Erfindung anhand von nichteinschränkenden Ausführungsbeispielen detaillierter beschrieben.

### BEISPIELE

In den folgenden Beispielen wurden Reaktionen gemäß dem folgenden allgemeinen Reaktionsschema durchgeführt:

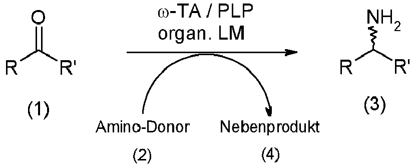

Auf diese Weise wurden mehrere Ketone (1) als prochirale Substrate unter enzymatischer Katalyse einer ω-Transaminase mit Pyridoxal-5'-phosphat (PLP) als Cofaktor in verschiedenen organischen Lösungsmitteln mit einem jeweiligen Wassergehalt unterhalb der Sättigungskonzentration zu den entsprechenden optisch aktiven Aminen (3) umgesetzt. Die ω-Transaminase überträgt dabei formal die Aminogruppe von einem Amino-Donor (2) auf das Keton (1), wobei derartige Enzyme üblicherweise über eine hohe Substratspezifität und Stereoselektivität verfügen. In der Regel wird eine Enantiomerenreinheit (Enantiomerenüberschuss, "enantiomeric excess", "e.e.") von über 99 % angestrebt.

Gleichzeitig wird der jeweilige Amino-Donor (2) zu einem Nebenprodukt (4) umgesetzt, bei dem es sich üblicherweise um ein Keton handelt.

Obgleich natürlich auch beliebige andere ω-Transaminasen einsetzbar sind, wurde als Enzym zu Vergleichszwecken durchwegs eine (R)-selektive ω-Transaminase von *Arthrobacter sp.* (kurz als ArR-ωTA bezeichnet) eingesetzt, da diese kostengünstig und leicht erhältlich ist und besonders hohe Stereoselektivität aufweist. Als Amino-Donor wurde entweder 2-Propylamin oder (R)-1-Phenylethylamin verwendet.

### Herstellung des Katalysators

### 1. Überexpression des Enzyms

Das Enzym wurde in E. *coli* unter Verwendung einer DNA-optimierter Gensequenz überexprimiert. LB-Amp-Medium (330 ml, 100 mg/l Ampicillin) wurde mit einer Zellsuspension (5 ml) aus einer Über-Nacht-Kultur beimpft, und die Zellen wurden bei 37 °C und 120 U/min kultiviert, bis die OD₆₀₀ 0,5 erreichte (nach etwa 3 h). Die Produktion der ω-Transaminase wurde durch Zusatz von Isopropylthiogalactopyranosid (IPTG, 0,5 mM) induziert. Die Kolben wurden über Nacht bei 120 U/min und 20 °C geschüttelt. Anschließend wurden die Zellen mittels Zentrifugation bei 12000 U/min und 4 °C über 20 min gewonnen. Das Pellet wurden mit Phosphatpuffer (pH 7, 100 mM, PLP 0,5 mM) gewaschen, mit flüssigem Stickstoff gefroren und lyophilisiert. Die so erhaltenen Zellen wurden bei 4 °C gelagert.

### 2. Zellaufschluss mittels Lysozym

Eine Stammlösung von EDTA (3,7 mg, 1 mM) und PLP (2,6 mg, 1 mM) wurde in Phosphatpuffer (10 ml, pH 7, 100 mM) bereitet. Eine Stammlösung von Phenylmethansulfonylfluorid (PMSF) (100 mM, 4,4 mg) wurde in 2-Propanol (250 µl) bereitet. Der Lysepuffer wurde unter Verwendung von Hühnereiweiß-Lysozym (10,6 mg, Sigma L6876, lyophilisiertes Pulver, 95 % Protein, > 40.000 U/mg Protein) im Phosphat/EDTA-Puffer (10 ml) bereitet, wonach schließlich PMSF (100 µl der 2-Propanol-Stammlösung) zugesetzt wurde. Lyophilisierte Zellen von E. *coli* BL21(DE3)/pET21-a-ω-TA (1 g) wurden im Lysepuffer (16 ml) suspendiert und auf einem Orbital Shaker mit 170 U/min bei Raumtemperatur 3 h lang geschüttelt. Anschließend wurde die Probe zentrifugiert (18000 U/min, 15 min, 4 °C), und der Überstand wurde mehrere Stunden lang lyophilisiert, was einen pulverförmigen Rohextrakt ergab, der ohne weitere Reinigung in den Transaminierungsreaktionen eingesetzt wurde. Dieses rohe Enzympräparat kann bei -20 °C mehrere Monate lang gelagert werden, ohne dass eine Abnahme der Aktivität festzustellen ist.

### 3. Zellaufschluss mittels French Press

E. coli-Zellen (2 g) aus Schritt 1 wurden in Phosphatpuffer (40 ml, pH 7, 100 mM, PLP 1 mM) resuspendiert und zweimal in einer French Press bei einem Druck von 12.000 psi und einer Temperatur von 4 °C aufgeschlossen. Die Zelltrümmer wurden abzentrifugiert (18.000 U/min, 15 min, 4 °C). Das klare Lysat des Überstands wurde lyophilisiert, was erneut einen pulverförmigen Rohextrakt ergab, der ohne weitere Reinigung in den Transaminierungsreaktionen eingesetzt wurde. Auch dieses rohe Enzympräparat kann bei -20 °C mehrere Monate lang gelagert werden, ohne dass eine Abnahme der Aktivität festzustellen ist.

### Bezugsbeispiele 1 bis 7- Äquilibrierungsversuche

Um die Angaben von Kim et al. (s.o.) zu verifizieren, wonach der Wassergehalt eines mit Wasser gesättigten organischen Lösungsmittels bei Äquilibrierung mit einem pulverförmigen, trockenen Enzympräprat sinkt, während jener von (nahezu) wasserfreiem Lösungsmittel steigt, da es zu einem Wasseraustausch zwischen dem Lösungsmittel und der festen Phase kommt, wurde die folgende Versuchsreihe durchgeführt. Ethylacetat (EtOAc) wurde über einem 3-Å-Molekularsieb getrocknet, bis ein mittels Karl-Fischer-Titration ermittelter Restwassergehalt von 11 ppm (entspricht 0,001 Vol.-%) erreicht war. Aus diesem "wasserfreien" EtOAc und destilliertem Wasser wurde eine Reihe von Lösungen von Wasser in EtOAc mit unterschiedlichen Konzentrationen unter der Sättigungskonzentration hergestellt. In jeweils 1 ml dieser Lösungen wurden in einer Glove-Box unter Argon 15 mg des obigen ω-TA-Enzym-Rohextrakts suspendiert und unter Luftausschluss 1 h lang geschüttelt. Der Wassergehalt des Lösungsmittels wurde danach erneut mittels Karl-Fischer-Titration bestimmt. Die Ergebnisse sind in nachstehender Tabelle 1 angegeben.

**Tabelle 1 - Wassergehalt von EtOAc vor und nach Äquilibrierung mit ω-TA**

| **Bezugsbeispiel Nr.** | **vor Äquilibrierung (Vol.-%)** | **nach Äquilibrierung (Vol.-%)** |
|---|---|---|
| 1 | 0,001 | 0,012 |
| 2 | 0,01 | 0,0184 |
| 3 | 0,1 | 0,0678 |
| 4 | 0,5 | 0,372 |
| 5 | 1,0 | 0,694 |
| 6 | 1,5 | 0,898 |
| 7 | 3,2 | 1,55 |

Aus der Tabelle ist klar zu erkennen, dass sich der Wassergehalt des Lösungsmittels bei der Äquilibrierung mit dem lyophilisierten Enzym ändert. In Lösungen von Wasser in EtOAc mit sehr niedriger Konzentration steigt diese an, in solchen mit höherer Konzentration (bis hin zur Sättigungsgrenze von 3,2 Vol.-%) sinkt sie hingegen. Ab 0,1 Vol.-% Wassergehalt ist für EtOAc ein Absinken der Wasserkonzentration aufgrund der Äquilibrierung zu beobachten. Das von Kim et al. (s.o.) festgestellte Verhalten wurde dadurch bestätigt.

### Beispiele 1 bis 11 - Aminierung in unterschiedlichen Lösungsmitteln

In diesen ersten Beispielen der Erfindung wurde die Wirkung verschiedener organischer Lösungsmittel auf die obige Aminierungsreaktion untersucht. Hierzu wurden die nachstehenden Vertreter unterschiedlicher Gruppen von Lösungsmitteln getestet.

### a) Wassermischbare organische Lösungsmittel

Dioxan, Dimethylsulfoxid (DMSO), Dimethylformamid (DMF), Acetonitril Hier wurde ein Wassergehalt von 1,7 Gew.-% eingestellt.

### b) Ether

Diisopropylether (DIPE), Methyl-tert-butylether (MTBE)

Diese Lösungsmittel wurden zunächst mit Wasser gesättigt, was (laut Literatur) Wasserkonzentrationen von 0,75 Gew.-% (DIPE) bzw. 1,03 Gew.-% (MTBE) ergibt.

### c) Ester

Ethylacetat (EtOAc)

Die Wassersättigungsgrenze liegt (laut Literatur) bei 3,2 Vol.-%, was bei einer Dichte von 0,894 g/cm³ etwa 3,7 Gew.-% entspricht.

### d) Aromatische Kohlenwasserstoffe

Toluol, Xylol (Isomerengemisch)

Die Sättigung mit Wasser ergibt (laut Literatur) Wasserkonzentrationen von 0,06 Gew.-% (Toluol) bzw. 0,05 Gew.-% (Xylol).

### e) Aliphatische Alkane

n-Pentan, n-Heptan

Die Sättigung mit Wasser ergibt (laut Literatur) Wasserkonzentrationen von 0,016 Gew.-% (n-Pentan) bzw. 0,013 Gew.-% (n-Heptan).

Jedes Lösungsmittel wurde danach 1 h lang mit einem wie oben beschriebenen erhaltenen pulverförmigen ω-Transaminase-Rohextrakt äquilibrieren gelassen, bevor Substrat und Amino-Donor zugesetzt wurden, wodurch der ursprüngliche Wassergehalt des Lösungsmittels durchwegs unter die Sättigungsgrenze sank (der in dieser Beispielsgruppe allerdings nicht näher bestimmt wurde).

Die Transaminierungsreaktionen wurden anschließend bei 30 °C unter 24-stündigem Schütteln bei 700 U/min in einem Eppendorf-Thermomischer durchgeführt. Neben der ArR-ω-TA (50 mM) und PLP als Cofaktor (1 mM) wurden Methoxyaceton (50 mM) als Substrat und (R)-1-Phenylethylamin (100 mM; 2 Äquiv.) als Amino-Donor eingesetzt. Als Produkt wurde daher (R)-1-Methoxy-2-propylamin und als Nebenprodukt Acetophenon erhalten.

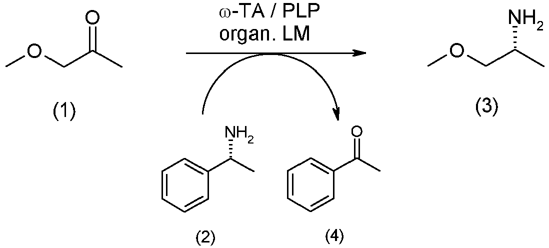

Die Ergebnisse bezüglich Reaktionsumsatz (basierend auf der Substratmenge) und Enantiomerenüberschuss (sofern dieser bestimmt wurde) sind in der nachstehenden Tabelle 2 angegeben.

**Tabelle 2 - Reaktionsumsatz und Enantiomerenüberschuss des chiralen Produkts**

| **Beispiel** | **Lösungsmittel** | **Umsatz (%)** | **e.e. (%)** |
|---|---|---|---|
| 1 | n-Pentan | 2 | n.b. |
| 2 | n-Heptan | 4 | n.b. |
| 3 | Toluol | 4 | n.b. |
| 4 | Xylol | 2 | n.b. |
| 5 | DIPE | 17 | n.b. |
| 6 | MTBE | 40 | >99 |
| 7 | EtOAc | 80 | >99 |
| 8 | Dioxan | 17 | >99 |
| 9 | DMSO | 0 | n.b. |
| 10 | DMF | 2 | n.b. |
| 11 | Acetonitril | 68 | >99 |

| | | | |
|---|---|---|---|
| n.b.: aufgrund geringer Ausbeute nicht bestimmt | | | |

Es zeigt sich, dass die Aminierung in allen Lösungsmitteln mit Ausnahme von DMSO mit ausgezeichneter Stereoselektivität durchführbar war, wenngleich erhebliche Unterschiede im Reaktionsumsatz zu beobachten waren. Diese Unterschiede lassen aber dennoch nicht notwendigerweise Aussagen über die prinzipielle Eignung der Lösungsmittel zu, da sich in der Folge gezeigt hat, dass der Umsatz in Abhängigkeit vom Wassergehalt des Lösungsmittels enorm schwankte.

### Beispiele 12 und 13 - Aminierung bei unterschiedlichen Wasserkonzentrationen

Aufgrund ihrer Verfügbarkeit und einfachen Handhabung wurden aus den zuvor getesteten Lösungsmitteln EtOAc und MTBE für weitere Versuche ausgewählt.

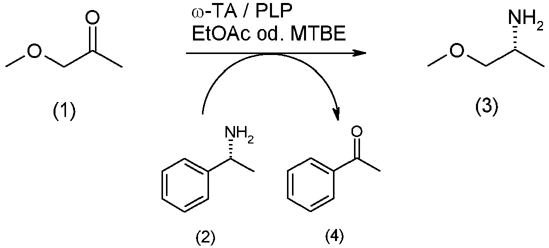

Die Reaktionen wurden jeweils in einer Reihe von Lösungsmittel/Wasser-Gemischen in unterschiedlichen Verhältnissen, die allesamt unter der jeweiligen Sättigungskonzentration lagen, analog zur Verfahrensführung in den obigen Beispielen (1 h Äquilibrierung, Verhältnis ω-TA:Amino-Donor = 1:2) durchgeführt, wobei jedoch zur Beschleunigung der Reaktionen die Temperatur auf 50 °C eingestellt wurde.

Die Sättigungskonzentration von Wasser in MTBE beträgt 1,03 Gew.-%, was mit einer Dichte von 0,74 g/cm³ 0,76 Vol.-% ergibt, und jene in EtOAc beträgt, wie oben erwähnt, 3,2 Vol.-%.

In EtOAc wurden Reaktionen bei Konzentrationen von unter 1,0 Vol.-% Wasser im Lösungsmittel aufgrund der niedrigen Umsatzraten 4 h lang laufen gelasssen, jene darüber hingegen nur 1 h lang. In MTBE wurden Reaktionen bei Wasserkonzentrationen unter 0,25 Vol.-% nach 2 h gestoppt, jene darüber schon nach 30 min. Der Reaktionsumsatz wurde anschließend jeweils mittels gaschromatographischer Analyse (GC) bestimmt.

In Fig. 1 sind die Ergebnisse dieser beiden Beispiele grafisch dargestellt, wobei die Testreihe für MTBE mittels voller Quadrate und einer durchgehenden Linie wiedergegeben ist und jene für EtOAc mittels voller Kreise und einer strichlierten Linie. Die Werte für den Wassergehalt sind durchwegs jene nach Äquilibrierung mit der ω-TA.

Es zeigte sich in beiden Fällen, dass die Reaktionsgeschwindigkeit, ausgedrückt als Umsatz pro Minute, bei zunehmendem Wassergehalt (in Vol.-%) ein Maximum erreichte, bei weiterer Erhöhung des Wassergehalts zur Sättigungsgrenze hin jedoch wieder abnahm. Dieses Maximum liegt für EtOAc bei etwa 2 Vol.-%, für MTBE bei etwa 0,65 Vol.-% und beträgt ca. 0,65 %/min in EtOAc und rund 1,5 %/min für MTBE. Diese Erkenntnis war vor dem Hintergrund der Publikation von Kim et al. (s.o.) besonders überraschend, zumal eine deren Schlussfolgerungen lautete, dass das Enzym umso aktiver sei, je mehr Wasser in der Hydrathülle um die Oberfläche des festen Präparats vorhanden ist. Erste Vorversuche mit weiteren organischen Lösungsmitteln bestätigten jedoch die Erkenntnis der vorliegenden Erfinder und ist Gegenstand derzeitiger Forschungen.

Von jeweils mehreren Reaktionsgemischen in EtOAc und MTBE wurden Proben gezogen, mittels derer die Enantiomerenreinheit des Produkts, d.h. 1-Methoxy-2-propylamin, untersucht wurde. Sie lag erneut durchwegs bei >99 %, wurde also weder durch den Wassergehalt noch durch die variierenden Reaktionsraten beeinflusst.

### Vergleichsbeispiel 1 - Transaminierung in wässrigem Puffer

Zum Vergleich der Reaktionsgeschwindigkeiten wurde dieselbe Reaktion wie in den Beispielen 12 und 13 in wässrigem Phosphatpuffer (pH = 7) wiederholt, wobei zunächst wiederum 50 mM, später sogar 100 mM ω-TA (1 mM PLP) eingesetzt wurden.

Überraschenderweise betrug die höchste dabei gemessene Reaktionsgeschwindigkeit, wiederum ausgedrückt als Umsatz pro Minute, nur 0,16 %/min. Somit betrugen die zuvor in den Beispielen 12 und 13 gemessenen maximalen Reaktionsgeschwindigkeiten von 0,65 %/min für EtOAc bzw. 1,5 %/min für MTBE das 4fache und sogar das 9,4fache jener in wässrigem Phosphatpuffer. Wie zuvor erwähnt hatten Kim et al. (s.o.) in wassergesättigtem EtOAc nur rund 30 % der Enzymaktivität von jener in wässrigem Phosphatpuffer feststellen können, was sich mit den vorliegenden Ergebnissen durchaus deckt, wenn man die strichlierte Linie aus Fig. 1 bis zu einem Wassergehalt von 3,2 Vol.-% extrapoliert.

### Beispiel 14 - Transaminierungen in MTBE mit 2-Propylamin als Amino-Donor

Zur Überprüfung, ob die obigen Reaktivitäten auch mit einem anderen Amino-Donor als (R)-1-Phenyl-2-propylamin erzielbar sind, wurde die obige Reaktion mit achiralem 2-Propylamin als Donor (2) bei 25 °C und mit dem in Beispiel 13 als optimal festgestellten Wassergehalt des Lösungsmittels von 0,65 Vol.-% durchgeführt.

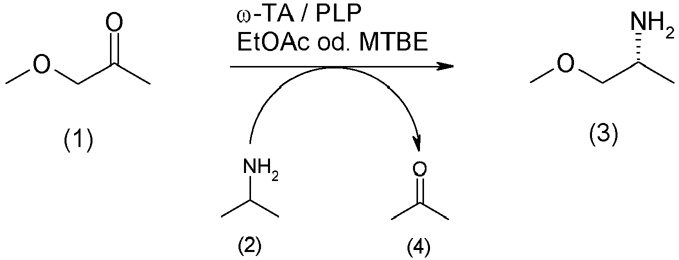

ArR-ω-TA: 20 mg, 0,68 U/mg;

PLP: 0,5 mM

Methoxyaceton: 50 mM;

2-Propylamin: 150 mM, 3 Äquiv.;

MTBE: 1 ml, 0,65 Vol.-% Wasser.

Die Enzymaktivität wurde in Phosphatpuffer (pH 7, 100 mM, 1 mM PLP) bei 30 °C unter Verwendung von Pyruvat (100 mM) als Substrat und von (R)-1-Phenylethyl-amin als Amino-Donor bestimmt, wobei 1 Unit Enzym pro Minute 1 µmol (R)-1-Phenylethylamin zu Acetophenon oxidiert.

Nach 10 min Reaktionszeit waren laut GC-Analyse schon über 50 % des Substrats (1) zum gewünschten Amin (3) umgesetzt, nach 1 h bereits rund 90 % und nach 2 h über 99 %. In einer Nebenreaktion wurde zwar durch Kondensation des Substrats (1) mit dem Amino-Donor (2) gleichzeitig eine Schiffsche Base gebildet:

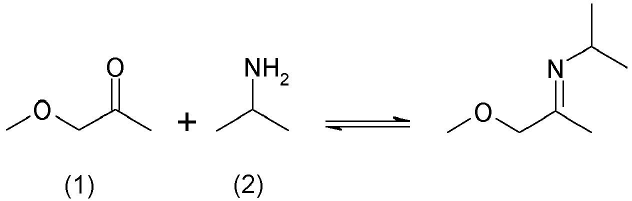

Diese Nebenreaktion ist jedoch reversibel, so dass bei längerer Reaktionszeit das gewünschte chirale Amin (3) dennoch quantitativ erhältlich war.

### Vergleichsbeispiel 2 - Transaminierung mit 2-Propylamin in wässrigem Puffer

Die obige Reaktion mit 2-Propylamin als Amino-Donor wurde unter ansonsten identischen Bedingungen in wässrigem Phosphatpuffer (pH = 7,5) wiederholt.

Nach 30 min und nach 1 h Reaktionszeit war im wässrigen Puffer keinerlei Umsatz feststellbar. Nach 2 h lag der Umsatz erst bei rund 1 %. Selbst nach 24 h waren nur 30 % des Ausgangsprodukts umgesetzt.

### Beispiele 15 bis 22 - Transaminierungen unterschiedlicher Substrate in MTBE

Zur Überprüfung, ob die obigen Beobachtungen nicht nur für Methoxyaceton als Substrat gelten, wurde eine Reihe weiterer Ketone der Transaminierungsreaktion gemäß Beispiel 14 in einem Eppendorf-Thermomischer mit 750 U/min bei einer Reaktionstemperatur von 25 °C unterzogen.

Enzym: ArR-ω-TA, 20 mg, 0,82 U/mg

Cofaktor: PLP, 0,5 mM

Substrat (1): 50 mM

Donor (2): 2-Propylamin, 150 mM, 3 Äquiv.

Lösungsmittel: MTBE, 0,65 Vol.-% Wassergehalt

In nachstehender Tabelle 3 sind die als Substrate eingesetzten Ketone sowie deren Reaktionsprodukte, d.s. die (*R*)-Isomere der entsprechenden Amine, angegeben. Um direkte Vergleiche zu ermöglichen, wurde in Beispiel 15 auch Methoxyaceton erneut untersucht.

**Tabelle 3 - Ketone als Substrate der Transaminierung**

| **Beispiel** | **Keton** | **Formel** | **Aminierungsprodukt** |
|---|---|---|---|
| 15 | Methoxyaceton | 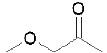 | 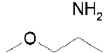 |
| 16 | Acetessigsäureethylester | 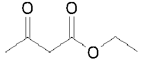 | 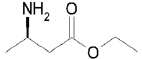 |
| 17 | o-Methoxyacetophenon | 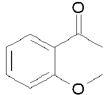 | 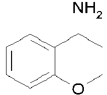 |
| 18 | α-Hydroxyacetophenon | 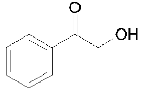 | 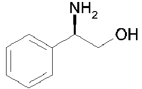 |
| 19 | Phenoxyaceton | 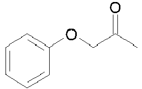 | 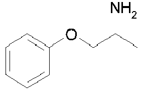 |
| 20 | Acetophenon | 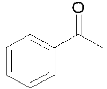 | 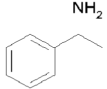 |
| 21 | Benzylaceton | 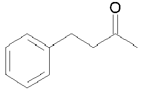 | 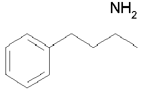 |
| 22 | 2-Octanon | 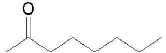 | 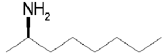 |

Aus den Reaktionsgemischen wurden in bestimmten Intervallen Proben gezogen, anhand derer erneut mittels GC die Umsätze bestimmt wurden. In nachstehender Tabelle 4 sind die gemessenen Ergebnisse für jedes Substrat aufgelistet, wobei der Enantiomerenüberschuss in allen acht Beispielen, also selbst bei geringem Umsatz, >99 % betrug.

**Tabelle 4 - Umsätze der Transaminierung**

| **Beispiel** | **Umsatz nach** | | | | | | |
|---|---|---|---|---|---|---|---|
| **Nr.** | **2h** | **4h** | **9h** | **13 h** | **24 h** | **36 h** | **72 h** |
| 15 | 98 | >99 | - | - | - | - | - |
| 16 | 46 | 66 | 88 | 94 | >99 | - | - |
| 17 | 0,1 | 0,4 | n.b. | n.b. | 2 | - | - |
| 18 | 62 | 80 | 95 | 97 | 98 | 98 | 98 |
| 19 | 70 | 87 | >99 | - | - | - | - |
| 20 | 3 | 7 | 13 | 19 | 29 | 38 | 43 |
| 21 | 3 | 5 | 9 | 12 | 23 | 32 | 48 |
| 22 | 2 | 3 | 7 | 11 | 18 | 27 | 41 |

Alle Substrate mit einem Sauerstoffatom oder einer Carbonylgruppe in β-Stellung zur Ketogruppe, d.h. jene der Beispiele 15, 16, 18 und 19, konnten durch die enzymatische Katalyse in organischem Lösungsmittel (nahezu) quantitativ in das entsprechende Amin übergeführt werden, und das sogar ohne Verschiebung des Reaktionsgleichgewichts durch Abzug des als Nebenprodukt (4) anfallenden Acetons (z.B. mittels Destillation).

Der äußerst geringe Umsatz von o-Methoxyacetophenon in Beispiel 17, speziell im Vergleich mit Acetophenon aus Beispiel 20, dürfte auf eine sterische Hinderung der Ketogruppe des Substrats durch den Methoxysubstituenten in Kombination mit der erhöhten Starrheit des Enzyms im organischen Lösungsmittel zurückzuführen sein.

Die Umsätze der Substrate der Beispiele 20 bis 22 (und vielleicht auch von Beispiel 17) könnten möglicherweise durch eine Erhöhung der Reaktionstemperatur gesteigert werden, wozu eventuell ein anderer Amino-Donor einzusetzen ist, da 2-Propylamin schon bei 32 °C siedet.

Andererseits war möglicherweise auch die Spezifität der verwendeten ω-Transaminase für manche der Substrate zu gering, weswegen in der Folge weitere Enyzme getestet wurden.

### Beispiele 23 bis 30 - Transaminierung mit anderen ω- Transaminasen

Die Beispiele 15 bis 22 wurden unter Verwendung derselben acht Ketone als Substrat, jedoch mit acht anderen, bis auf eine Ausnahme nun allesamt (S)-selektiven ω-Transaminasen bei ansonsten identischen Bedingungen wiederholt. Die Produkte (3) der Aminierungsreaktionen waren in den Beispielen 23 bis 29 logischerweise die entsprechenden (S)-Isomere der in Tabelle 3 angeführten Amine, allerdings unter Ausnahme von Beispiel 26. Aufgrund des Hydroxymethyl-Substituenten, der gemäß Cahn-Ingold-Prelog-Regeln eine höhere Priorität besitzt als Phenyl, erzeugt eine (S)-selektive ω-Transaminase, die daher auch (S)-1-Phenylethylamin oder andere (S)-Amine als Amino-Donoren nutzt, aus α-Hydroxyacetophenon ebenfalls das (R)-Isomer von 2-Hydroxy-1-phenylethylamin.

Die acht eingesetzten ω-Transaminasen waren die folgenden:
- CV:: ω-TA von *Chromobacterium violaceum,* 2,04 U/mg
- PF:: ω-TA von *Pseudomonas fluorescens,* 0,26 U/mg
- His-VF:: His-markierte ω-TA von *Vibrio fluvialis,* 2,92 U/mg
- Strp-PD:: Strep-markierte ω-TA von *Paracoccus denitrificans,* 0,95 U/mg
- BM:: ω-TA von *Bacillus megaterium,* 0,51 U/mg
- His-ArS:: His-markierte (S)-selektive ω-TA von *Arthrobactercitreus,* 0,74 U/mg
- AD:: ω-TA von *Alcaligenes denitrificans,* 0,35 U/mg
- ArRmut11:: ω-TA der (R)-selektiven Mutante-11 von *Arthrobacter sp.*

Die Aktivität wurde in Phosphatpuffer (pH 7, 100 mM, 1 mM PLP) bei 30 °C unter Verwendung von Pyruvat (100 mM) als Substrat und von (S)-1-Phenylethylamin bzw. (S)-1-Methoxy-2-propylamin als Amino-Donor bestimmt, wobei 1 Unit Enzym pro Minute 1 µmol (S)-1-Phenylethylamin zu Acetophenon bzw. 1 µmol (*S*)-1-Methoxy-2-propylamin zu Methoxyaceton oxidiert. Die Aktivität des (R)-selektiven Enzyms aus Beispiel 30 konnte jedoch nicht bestimmt werden, da diese *Arthrobacter-*Mutante Pyruvat nicht als Substrat akzeptiert.

Die Substrate waren dieselben wie in den Beispielen 15 bis 22, nämlich:
Beispiel 23 - Methoxyaceton
Beispiel 24 - Acetessigsäureethylester
Beispiel 25 - o-Methoxyacetophenon
Beispiel 26 - α-Hydroxyacetophenon
Beispiel 27 - Phenoxyaceton
Beispiel 28 - Acetophenon
Beispiel 29 - Benzylaceton
Beispiel 30 - 2-Octanon

In nachstehender Tabelle 5 sind die Ergebnisse der Transaminierungen aufgelistet, wobei "C" ("conversion") für den Umsatz nach der verstrichenen Reaktionszeit und "ee" für den Enantiomerenüberschuss des dabei erhaltenen (S)- oder (R)-Isomers steht.

Die für das jeweilige Substrat besten Ergebnisse sind durch Fettdruck hervorgehoben, wobei in den Beispielen 27 und 30 durch Verlängerung der Reaktionszeit zwar eine Erhöhung des Umsatzes erzielbar war, das Verfahren dadurch aber möglicherweise zu unwirtschaftlich würde. In diesen Fällen sind zwei Ergebnisse fett gedruckt.

**Tabelle 5 - Transaminierungen der Substrate mit unterschiedlichen Enzymen**

| **Beispiel Nr.** | **Zeit (h)** | **Enzym** | | | | | | | | | | | | | | | |
|---|---|---|---|---|---|---|---|---|---|---|---|---|---|---|---|---|---|
| | | **CV** | | **PF** | | **His-VF** | | **Strp-PD** | | **BM** | | **His-ArS** | | **AD** | | **ArR-mut11** | |
| | | **C** | **ee** | **C** | **ee** | **C** | **ee** | **C** | **ee** | **C** | **ee** | **C** | **ee** | **C** | **ee** | **C** | **ee** |
| | | **(%)** | **(S)** | **(%)** | **(S)** | **(%)** | **(S)** | **(%)** | **(S)** | **(%)** | **(S)** | **(%)** | **(S)** | **(%)** | **(S)** | **(%)** | **(R)** |
| 23 | 2 | 95 | 92 | <1 | 98 | 45 | 40 | 3 | 99 | 89 | >99 | 98 | >99 | <1 | - | 65 | 94 |
| | 4 | 98 | - | 3 | - | 69 | - | 13 | - | 94 | - | >99 | >99 | <1 | - | 84 | - |
| | 12 | >99 | - | 20 | - | 94 | - | 38 | - | >99 | - | >99 | - | <1 | - | >99 | - |
| | 24 | >99 | 92 | 31 | 98 | >99 | 40 | 52 | 96 | >99 | >99 | >99 | >99 | <1 | - | >99 | 92 |
| 24 | 4 | 30 | 96 | <1 | - | 35 | 98 | 7 | 99 | 9 | >99 | 53 | >99 | <1 | - | 18 | 98 |
| | 12 | 63 | - | <1 | - | 60 | - | 14 | - | 8 | - | 88 | >99 | <1 | - | 50 | - |
| | 24 | 72 | - | <1 | - | 74 | - | 17 | - | 6 | - | 84 | - | <1 | - | 68 | - |
| | 72 | 65 | 94 | - | - | 67 | 96 | 24 | 96 | 4 | >99 | 74 | >99 | <1 | - | 70 | 94 |
| 25 | 4 | <1 | - | <1 | - | <1 | - | <1 | - | 4 | - | <1 | - | <1 | - | <1 | - |
| | 12 | 1 | - | <1 | - | <1 | - | <1 | - | 5 | - | 1 | - | <1 | - | <1 | - |
| | 24 | 2 | - | <1 | - | 1 | - | <1 | - | 4 | - | 2 | - | <1 | - | 1 | - |
| | 72 | 4 | >99 | <1 | - | 4 | >99 | 1 | - | 11 | >99 | 6 | >99 | <1 | - | 6 | >99 |
| 26 | 4 | 1 | - | 1 | - | 1 | - | 1 | 1 | **-** | - | - | - | - | - | - | - |
| | 8 | 2 | - | 1 | - | 1 | - | 1 | 1 | - | - | 1 | - | 1 | - | 2 | |
| | 12 | 3 | - | 1 | - | 1 | - | 1 | - | 2 | - | 2 | - | 1 | - | 3 | - |
| | 24 | 5 | - | 1 | - | 1 | - | 1 | - | 1 | - | 3 | - | 1 | - | 6 | - |
| | 72 | 7 | >99* | 1 | - | 1 | - | 1 | - | 1 | - | 4 | >99* | 1 | - | 11 | >99* |
| 27 | 4 | 83 | - | 2 | - | 45 | - | 4 | - | 49 | - | 49 | - | <1 | - | 35 | - |
| | 8 | 95 | - | 4 | - | 68 | - | 9 | - | 68 | - | 69 | - | <1 | - | 60 | - |
| | 12 | 97 | >99 | 6 | - | 80 | - | 11 | - | 78 | - | 81 | - | <1 | - | 76 | - |
| | 24 | >99 | >99 | 12 | - | 91 | 96 | 23 | 98 | 88 | >99 | 92 | >99 | <1 | - | 91 | 94 |
| | 72 | >99 | >99 | 29 | >99 | 98 | 96 | 46 | 98 | 94 | 99 | >99 | >99 | <1 | - | 98 | 91 |
| 28 | 24 | 5 | - | <1 | - | 2 | - | <1 | - | <1 | - | 20 | - | <1 | - | 10 | - |
| | 72 | 9 | >99 | <1 | - | 4 | >99 | 1 | - | 1 | - | 39 | >99 | <1 | - | 23 | >99 |
| 29 | 24 | 3 | >99 | <1 | - | 3 | 87 | <1 | - | <1 | - | 48 | >99 | <1 | - | 14 | 95 |
| | 72 | 16 | 99 | <1 | - | 5 | 85 | <1 | - | <1 | - | 53 | 98 | <1 | - | 32 | 89 |
| 30 | 24 | <1 | - | <1 | - | 1 | - | <1 | - | <1 | - | 38 | >99 | <1 | - | 4 | 97 |
| | 72 | <1 | - | <1 | - | 1 | - | <1 | - | <1 | - | 44 | >99 | <1 | - | 14 | - |

| | | | | | | | | | | | | | | | | | |
|---|---|---|---|---|---|---|---|---|---|---|---|---|---|---|---|---|---|
| * In Beispiel 26 wurde, wie oben erwähnt, durchwegs das (R)-Isomer erhalten. | | | | | | | | | | | | | | | | | |

Die zuvor in den Beispielen 15, 16 und 19 unter Verwendung von ArR-ω-TA rasch, quantitativ und mit ausgezeichneter Enantiomerenreinheit transaminierbaren Substrate, d.h. Methoxyaceton, Acetessigsäureethylester und Phenoxyacetophenon, stellten auch für andere Enzyme geeignete Substrate dar, wie die Beispiele 23, 24 und 27 belegen.

Im Gegensatz dazu konnte α-Hydroxyacetophenon, das zuvor in Beispiel 18 gut umsetzbar gewesen war, in Beispiel 26 mit keinem Enzym zufrieden stellend aminiert werden (maximaler Umsatz 7 % mit CV-ωTA). Dasselbe gilt auch für o-Methoxyacetophenon in Beispiel 25, das wie auch schon davor in Beispiel 17 kaum umsetzbar war. Immerhin konnte der Umsatz im Fall von BM-ωTA auf 11 % (nach 72 h) erhöht werden (zuvor mit ArR-ωTA waren es 2 % nach 24 h). Das spricht erneut für eine sterische Hinderung der Ketogruppe durch den Methoxysubstituenten am Phenylring.

Die Substrate ohne zusätzlichen Sauerstoff, d.h. Acetophenon, Benzylaceton und 2-Octanon, der Beispiele 28 bis 30 waren nur mit His-ArS-ωTA einigermaßen gut zu aminieren, d.h. mit Umsätzen im Bereich von rund 40-50 %, was sich mit den Ergebnissen der Beispiele 20 bis 22 mit ArR-ωTA deckt. Allerdings konnten diese Umsätze mit His-ArS-ωTA zum Teil schon nach 24 h und nicht erst nach 72 h erzielt werden.

Dies bestätigt, dass für die erfindungsgemäße Transaminierung in organischem Lösungsmittel jene Ketone als Substrate zu bevorzugen sind, die über eine "aktivierte" Ketogruppe verfügen, d.h. Sauerstoff oder einen sauerstoffhaltigen Substituenten in β-Stellung zur Carbonylgruppe aufweisen. Wechselwirkungen mit anderen Heteroatomen, wie z.B. Stickstoff oder Schwefel, könnten allerdings Ähnliches bewirken und werden daher Gegenstand weiterer Untersuchungen sein.

Mitunter kam es bei Verlängerung der Reaktionszeit zu einer Verschlechterung der Enantiomerenreinheit, was zeigt, dass dies nicht notwendigerweise ein geeignetes Mittel zur Umsatzsteigerung darstellt.

### Beispiele 31 bis 35 - Transaminierung mit höheren Mengen an Amino-Donor

Exemplarisch für die drei Substrate ohne zusätzlichen Sauerstoff wurde Benzylaceton (4-Phenyl-2-butanon), das in den Beispielen 21 und 29 mit mittleren Umsätzen von knapp 50 % (nach 72 bzw. 24 h Reaktionszeit) aminiert worden war, nun erneut mit ArR-ωTA (20 mg, 0,74 U/mg) in einer Substratkonzentration von 50 mM in MTBE mit 0,65 Vol.-% Wassergehalt bei 25 °C umgesetzt. Die Konzentration des Amino-Donors 2-Propylamin, wurde dabei jedoch zwischen 150 mM (3 Äquiv.) und 1 M (20 Äquiv.) variiert. Die Ergebnisse sind in nachstehender Tabelle 6 aufgelistet, wobei in allen Fällen ein Enantiomerenüberschuss von >99 % erzielt wurde.

**Tabelle 6 - Transaminierung von Benzylaceton mit variierender Donormenge**

| **Beispiel** | **Molverhältnis** | **Umsatz (%)** | | |
|---|---|---|---|---|
| **Nr.** | **Substrat : Donor** | **nach 24 h** | **nach 48 h** | **nach 120 h** |
| 31 | 1:3 | 44 | 50 | 51 |
| 32 | 1:5 | 49 | 64 | 68 |
| **33** | **1:10** | **45** | **66** | **82** |
| 34 | 1:15 | 37 | 57 | 79 |
| 35 | 1:20 | 30 | 47 | 68 |

Beispiel 31 entspricht einer Wiederholung von Beispiel 21 und lieferte auch ein vergleichbares Ergebnis, nämlich einen Umsatz von rund 50 %, wenn auch in diesem Fall schon nach kürzerer Reaktionszeit. Das beste Ergebnis von 82 % Umsatz (wiederum fettgedruckt) wurde in Beispiel 33 mit 10 Äquivalenten Amino-Donor nach 5 d Reaktionszeit erzielt, ohne dass sich dadurch der Enantiomerenüberschuss verschlechterte. Diese Versuchsreihe zeigt somit, dass auch Substrate ohne zusätzlichen Sauerstoff-Substituenten nach dem erfindungsgemäßen Verfahren mit gutem Umsatz transaminiert werden können, wenn auch lange Reaktionszeiten erforderlich sind. Diese könnten gegebenenfalls durch Erhöhung der Menge oder auch der Aktivität des Enzyms (z.B. mittels "enzyme engineering") verkürzt werden.

### Beispiele 36 bis 41 - Transaminierung mit höheren Mengen an Enzym

Zur Überprüfung, ob eine höhere Enzymaktivität im Reaktionsgemisch bessere Umsätze bewirken würde, wurde die Menge an ArR-ωTA gegenüber den Beispielen 31 bis 35 auf 40 mg bzw. 80 mg verdoppelt bzw. vervierfacht und die Menge an Amino-Donor zwischen 10 und 20 Äquivalenten variiert. Die Ergebnisse sind in Tabelle 7 aufgelistet, wobei wiederum in allen Fällen >99 % e.e. erzielt wurden.

**Tabelle 7 - Transaminierung von Benzylaceton mit höherer Katalysatormenge**

| **Beispiel** | **Enzym (mg)** | **Molverhältnis** | **Umsatz (%)** | | |
|---|---|---|---|---|---|
| **Nr.** | | **Substrat : Donor** | **nach 24 h** | **nach 48 h** | **nach 72 h** |
| 36 | 40 | 1 : 10 | 65 | 73 | 74 |
| 37 | 40 | 1 : 15 | 59 | 77 | 83 |
| 38 | 40 | 1 : 20 | 39 | 57 | 66 |
| 39 | 80 | 1 : 10 | 65 | 65 | 66 |
| 40 | 80 | 1 : 15 | **75** | 81 | 82 |
| 41 | 80 | 1 : 20 | 65 | 81 | **86** |

Wie erneut mittels Fettdruck gekennzeichnet ist, sind besonders zwei Ergebnisse vorteilhaft. Einerseits konnte in Beispiel 41 mit der vierfachen Menge an Enzym und 20 Äquivalenten Amino-Donor nach 72 h Reaktionszeit ein Umsatz von 86 % erzielt werden, was eine sehr geringfügige Verbesserung gegenüber Beispiel 33 darstellt, Anderseits jedoch wurde in Beispiel 40 mit der vierfachen Menge an Enzym und 15 Äquivalenten Amino-Donor schon nach 24 h Reaktionszeit ein Umsatz von 75 % erreicht, was die wirtschaftlich interessantere Variante der Verfahrensführung darstellen dürfte, obwohl sich auch in dieser Versuchsreihe der Enantiomerenüberschuss mit zunehmender Reaktionszeit nicht verschlechterte.

### Beispiel 42 - Transaminierung mit rezykliertem Enzym

Einer der Vorteile der Biokatalyse in organischen Lösungsmitteln liegt darin, dass die Enzympräparate, z.B. durch einfache Filtration, leicht vom Reaktionsgemisch abtrennbar und darüber hinaus nach der Abtrennung erneut einsetzbar sind. Zur Überprüfung dieses Umstands wurde in einer Modellreaktionsreihe wiederum Methoxyaceton (50 mM) in MTBE (1 ml; 0,65 Vol.-% Wasser) bei 25 °C mit 2-Propylamin als Donor (150 mM, 3 Äquiv.) in einem Eppendorf-Thermomischer (750 U/min) mit ArR-ωTA (roher Zellextrakt, 20 mg, 0,82 U/mg) umgesetzt.

Nach jeweils 4 h Reaktionszeit wurde das Enzym abfiltriert, mit trockenem MTBE gewaschen und im nächsten Reaktionsansatz als Katalysator eingesetzt. Auf diese Weise wurde ein und dasselbe Enzympräparat in insgesamt 10 jeweils vierstündigen Transaminierungsreaktionen (R1 bis R10) eingesetzt. Die auf diese Weise in drei parallelen Ansätzen erzielten Umsätze sind in Tabelle 8 angeführt.

**Tabelle 8 - Umsatz von Methoxyaceton mit rezykliertem Enzympräparat**

| **Ansatz** | **Umsatz (%)** | | | | | | | | | |
|---|---|---|---|---|---|---|---|---|---|---|
| **Nr.** | **R1** | **R2** | **R3** | **R4** | **R5** | **R6** | **R7** | **R8** | **R9** | **R10** |
| 1 | >99 | >99 | >99 | 99 | 99 | 90 | 77 | 68 | 58 | 50 |
| 2 | >99 | >99 | >99 | 98 | 97 | 80 | 63 | 53 | 43 | 39 |
| 3 | >99 | >99 | 99 | 98 | 97 | 77 | 59 | 50 | 40 | 37 |

Man erkennt, dass das Enzym in bis zu 5 aufeinander folgenden Reaktionsansätzen (wieder-)verwendet werden kann, ohne dass der Umsatz unter 99 % sinkt (Ansatz 1). Obwohl spätestens ab der 6. Reaktion die Enzymaktivität merklich abnimmt, konnten in der 10. Reaktion noch immer 50 % Umsatz erzielt werden.

Somit reicht es nicht nur aus, rohen, lyophilisierten Zellextrakt als Enzympräparat im Verfahren der vorliegenden Erfindung einzusetzen, das Enzym ist darüber hinaus sogar mehrfach rezyklierbar.

Die obigen Beispiele belegen jedenfalls eindeutig, dass gemäß vorliegender Erfindung prochirale Ketone unter enzymatischer Katalyse mit ω-Transaminasen in organischen Lösungsmitteln, die einen Wassergehalt unterhalb der Sättigungskonzentration aufweisen, mit guten Ausbeuten und hoher Enantiomerenreinheit in die entsprechenden chiralen Amine übergeführt werden können. Die mit dem Verfahren der Erfindung erzielbaren Umsätze sind mitunter höher als jene im jeweiligen wassergesättigten Lösungsmittel und bisweilen sogar höher als jene in wässrigen Puffersystemen.

## Patentansprüche

1. Verfahren zur Herstellung eines Amins (3) durch Aminierung eines Ketons (1) mittels enzymatischer Katalyse unter Verwendung einer ω-Transaminase (ω-TA) als Katalysator, von Pyridoxal-5'-phosphat (PLP) als Cofaktor und eines Amins (2) als Amino-Donor, wobei die nachstehende Reaktion in einem organischen Lösungsmittel durchgeführt wird: 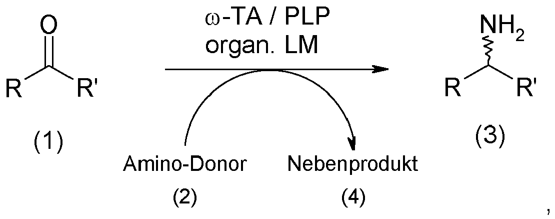 worin R und R' einwertige Kohlenwasserstoffreste sind,
**dadurch gekennzeichnet, dass** im organischen Lösungsmittel eine zwischen 0 und der Sättigungskonzentration cₛ des jeweiligen Lösungsmittels liegende Wasserkonzentration c_{w} eingestellt wird, bevor die Aminierungsreaktion durchgeführt wird, so dass gilt: 0 < c_{w} < cₛ.

2. Verfahren nach Anspruch 1, **dadurch gekennzeichnet, dass** R und R' aus der aus gegebenenfalls substituierten Alkyl-, Aryl-, Arylalkyl- und Alkylaryl-Resten bestehenden Gruppe ausgewählt sind.

3. Verfahren nach Anspruch 2, **dadurch gekennzeichnet, dass** R und/oder R' mit einer oder mehreren Hydroxy-, Oxo-, Carbonyl- und/oder Carboxylgruppen substituiert sind und/oder ein oder mehrere Kohlenstoffatome durch Heteroatome, insbesondere Sauerstoffatome, ersetzt sind.

4. Verfahren nach Anspruch 3, **dadurch gekennzeichnet, dass** zumindest einer von R und R' die Struktur -CH₂-O-R" oder -CH₂-(C=O)-R" aufweist, worin R" ein Wasserstoffatom oder ein einwertiger Kohlenwasserstoffrest wie in Anspruch 2 oder 3 definiert ist.

5. Verfahren nach einem der vorangegangenen Ansprüche, **dadurch gekennzeichnet, dass** eine Äquilibrierung der ω-Transaminase im Lösungsmittel durchgeführt wird, bis sich eine konstante Wasserkonzentration im Lösungsmittel eingestellt hat.

6. Verfahren nach einem der vorangegangenen Ansprüche, **dadurch gekennzeichnet, dass** die Wasserkonzentration durch Zudosieren von zusätzlicher ω-Transaminase, wasserfreiem Lösungsmittel oder Wasser zu einem Lösungsmittel und ω-Transaminase umfassenden Gemisch eingestellt wird.

7. Verfahren nach einem der vorangegangenen Ansprüche, **dadurch gekennzeichnet, dass** als ω-Transaminase ein lyophilisierter Rohextrakt von das Enzym produzierenden Zellen eingesetzt wird.

8. Verfahren nach einem der vorangegangenen Ansprüche, **dadurch gekennzeichnet, dass** eine ω-Transaminase (ω-TA) eingesetzt wird, die aus der aus den folgenden Enzymen bestehenden Gruppe ausgewählt ist: der (R)-selektiven ω-Transaminase von *Arthrobacter sp.,* der ω-TA von *Chromobacterium violaceum,* der ω-TA von *Pseudomonas fluorescens,* der His-markierten ω-TA von *Vibrio fluvialis,* der Strep-markierten ω-TA von *Paracoccus denitrificans,* der ω-TA von *Bacillus megaterium,* der His-markierten, (S)-selektiven ω-TA von *Arthrobacter citreus,* der ω-TA von *Alcaligenes denitrificans* und der (R)-selektiven ω-TA der Mutante 11 von *Arthrobacter sp.*

9. Verfahren nach Anspruch 8, **dadurch gekennzeichnet, dass** die (R)-selektive ω-Transaminase von *Arthrobacter sp.* eingesetzt wird.

10. Verfahren nach einem der vorangegangenen Ansprüche, **dadurch gekennzeichnet, dass** die ω-Transaminase an einen festen Träger gebunden ist.

11. Verfahren nach einem der vorangegangenen Ansprüche, **dadurch gekennzeichnet, dass** ein aus Methyl-tert-butylether, Ethylacetat, Acetonitril, Diisopropylether, Dioxan und Gemischen davon ausgewähltes organisches Lösungsmittel eingesetzt wird.

12. Verfahren nach Anspruch 11, **dadurch gekennzeichnet, dass** als Lösungsmittel Methyl-tert-butylether, Ethylacetat oder Acetonitril eingesetzt wird.
